(19) Europäisches Patentamt
European Patent Office
Office européen des brevets

(11) **EP 1 278 503 B1**

(12) **EUROPEAN PATENT SPECIFICATION**

(45) Date of publication and mention
of the grant of the patent:
**13.08.2008 Bulletin 2008/33**

(51) Int Cl.:
*A61K 6/083* (2006.01) *A61K 6/00* (2006.01)

(21) Application number: **00928730.1**

(22) Date of filing: **03.05.2000**

(86) International application number:
**PCT/US2000/011938**

(87) International publication number:
**WO 2001/082876 (08.11.2001 Gazette 2001/45)**

(54) **FLUOROPOLYMERIC ORTHODONTIC BRACKETS**

ORTHODONTISCHE SCHIENEN AUS FLUOROPOLYMER

APPAREIL DENTAIRE EN FLUOROPOLYMERE

(84) Designated Contracting States:
**AT BE CH CY DE DK ES FI FR GB GR IE IT LI LU MC NL PT SE**

(43) Date of publication of application:
**29.01.2003 Bulletin 2003/05**

(73) Proprietor: **3M Innovative Properties Company**
**St. Paul MN 55133-3427 (US)**

(72) Inventors:
• **HORN, Jerold, S.**
**Saint Paul, MN 55133-3427 (US)**

• **HANSEN, James, D.**
**Saint Paul, MN 55133-3427 (US)**

(74) Representative: **Vossius & Partner**
**Siebertstrasse 4**
**81675 München (DE)**

(56) References cited:
**WO-A-95/22567**      **GB-A- 2 253 420**
**US-A- 3 545 083**

**Description**

This invention relates to fluoropolymeric orthodontic brackets.

Orthodontic treatment often involves the application of mechanical forces to urge improperly positioned teeth into correct alignment. One common form of orthodontic treatment includes the use of orthodontic brackets affixed to the surface of a tooth, and a resilient curved arch wire seated in slots on the brackets. The arch wire exerts a restoring force on the teeth which tends to shift the teeth into orthodontically correct alignment.

Orthodontic articles such as, e.g., brackets, were traditionally formed from metal, but more recently have been formed from plastic and ceramic. Plastic brackets can be fabricated to be translucent to transparent in character relative to metal brackets. It is often difficult, however, to maintain the aesthetic characteristics of plastic brackets during use because food and beverages can stain and discolor the brackets while they reside in a patient's mouth.

GB-A-2 253 420 relates to a process for the production of a homogeneous polymeric monolith characterised in that an assembly of oriented polymer fibres is maintained in intimate contact at an elevated temperature sufficient to melt a proportion of the polymer and is subsequently compressed.

US-A-3,545,083 describes a dental application of a special polymer of tetrafluoroethylene tubing for masking and cushioning metal clasps and orthodontic wires.

WO 95/22567 relates to a polymerizable acrylic composition which comprises: (a) at least one acrylic monomer; (b) an effective amount of a specific organoborane amine complex and (c) an effective amount of an acid for initiating polymerization of the acrylic monomer.

EP-A-498 558 discloses an elastomeric orthodontic device that is sufficiently free of hydrocarbon-based polyether segments and hydrocarbon-based polyester segments to provide resistance to staining, said device being made of a material having per 2 mm thickness a Hunter color difference when using a 2.5 cm illuminated opening of (a) less than 40 when compared to a Hunter white standard tile and (b) less than 33 after immersion in mustard solution for 30 minutes when compared to the material before immersion in mustard solution.

In one aspect, the invention features an orthodontic bracket comprising a fluoropolymer selected from the group consisting of perfluoroethylene-propylene copolymer, perfluoroalkoxyethylene, ethylene-tetrafluoroethylene copolymer, polychlorotrifluoroethylene, ethylene-chlorotrifluoroethylene copolymer, or a combination thereof.

In one embodiment, the article exhibits a Delta E color shift of no greater than about 2 when tested according to the Hydrophilic Color Shift Test, and a Delta E color shift of no greater than about 5 when tested according to the Oleophilic Color Shift Test.

In another embodiment, the fluoropolymer includes perfluoroethylene-propylene copolymer. In another embodiment, the fluoropolymer includes perfluoroalkoxyethylene. In other embodiments, the fluoropolymer includes ethylene-chlorotrifluoroethylene copolymer.

The orthodontic bracket may further include a polymeric composition disposed on a surface of said bracket, where the polymeric composition includes an organoborane compound. In another embodiment, the bracket further includes an organoborane amine complex disposed on a surface of the bracket. The orthodontic bracket may further include a metallic component.

In another aspect, the invention features a kit for adhering an orthodontic bracket to a tooth. The kit includes an orthodontic bracket as defined above; and an adhesive system comprising a) a polymerizable component, and b) and organoborane amine complex.

In another embodiment, the kit further includes a polyimide film.

The orthodontic brackets resistant to staining by food and beverages such as mustard and spaghetti sauce such that the bracket remains aesthetically pleasing in appearance and does not unduly darken or turn yellow during the time the bracket remains in the oral cavity. The orthodontic brackets also maintain their essentially translucent to transparent character over the useful life of the bracket such that they transmit the color of the underlying tooth surface to which they are adhered.

Further details of the invention are defined in the features of the claims.

FIG. 1 is a front view looking toward a buccolabial side (i.e., toward a lip or cheek facing side) of an orthodontic bracket;
FIG. 2 is an end view of the orthodontic bracket of FIG. 1;
FIG. 3 is a front view looking toward a buccolabial side (i.e:, toward a lip or cheek facing side) of a second embodiment of an orthodontic bracket;
FIG. 4 is an end view of the orthodontic bracket of FIG. 3;
FIG. 5 is a front view of a third embodiment of a test article;
FIG. 6 is a perspective side view of the test article of FIG. 5;
FIG. 7 is a plot of % transmittance versus wavelength (nm) for the bracket of Example 12;
FIG. 8 is a plot of % transmittance versus wavelength (nm) for the bracket of Example 13;
FIG. 9 is a plot of % transmittance versus wavelength (nm) for the bracket of Example 14;

FIG. 10 is a plot of % transmittance versus wavelength (nm) for the bracket of Example 15;

FIG. 11 is a plot of % transmittance versus wavelength (nm) for the bracket of Example 16;

FIG. 12 is a plot of % transmittance versus wavelength (nm) for the bracket of . Comparative Example 1;

FIG. 13 is a front view looking toward a buccolabial side of an embodiment of an orthodontic bracket that includes a metallic framework;

FIG. 14 is a front view looking toward a buccolabial side of another embodiment of an orthodontic bracket that includes a metallic framework; and

FIG. 15 is a perspective view of the metallic framework of FIG. 14.

FIG. 16 is a side view of another embodiment of an orthodontic bracket that includes a fluoropolymeric layer on a nonfluoropolymeric interior.

Orthodontic brackets are capable of being mounted on a tooth, and are used to transmit to the tooth a corrective force from an arch wire, spring, elastic, or other activatible force-applying component.

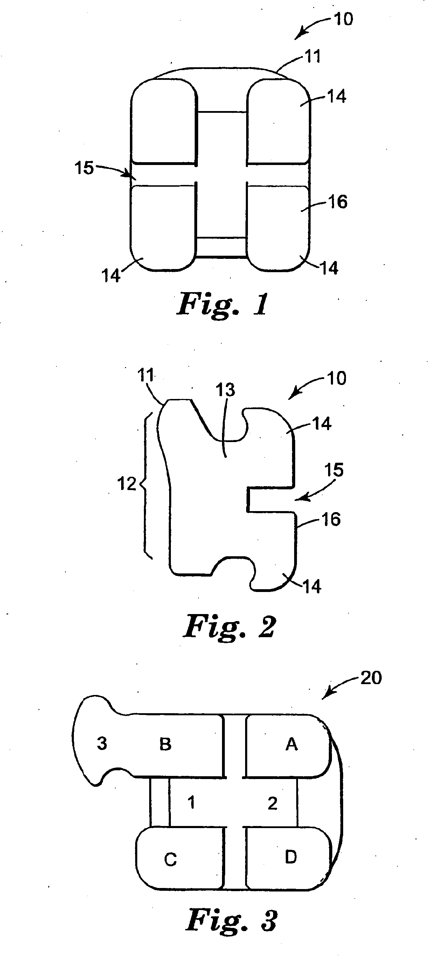

FIGS. 1 and 2 show an exemplary orthodontic bracket 10. The bracket has a base 11 suitable for either direct bonding to a tooth or attachment to any kind of mounting fixture. A tooth facing surface 12 of the base 11 can be concavely curved about both mesiodistal axis and an apical axis to match the natural convexity of the tooth labial surface, but other curvatures or flat surfaces can also be used to accommodate bracket positioning. A bracket body 13 extends from base 11 to define bracket tie-wings 14 for ligature anchorage, and a mesiodistally oriented arch-wire slot 15 extending from an outer body surface 16 into the bracket body.

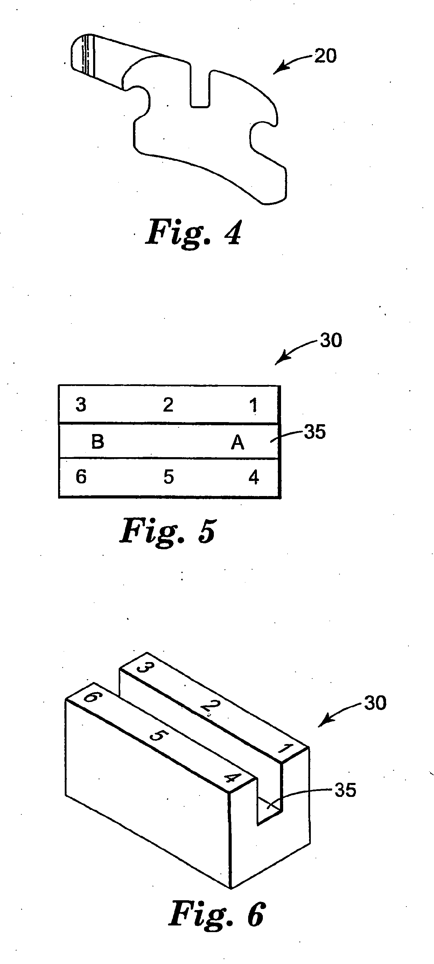

FIGS. 3 and 4 depict a second embodiment of an orthodontic bracket 20 as seen from the buccolabial side and from the end, respectively. FIGS. 5 and 6 depict a test article 30 in which a slot 35 is seen from the top in FIG. 5. The article is shown in perspective side view in FIG. 6. The test article of FIGS. 5 and 6 approximates the overall size and thickness of a conventional orthodontic bracket.

The fluoropolymeric orthodontic brackets described herein are resistant to staining during use such that the brackets maintain their original color as determined by the unaided human eye under common lighting conditions (e.g., sunlight and incandescent light). The fluoropolymeric orthodontic brackets are resistant to staining from oil-based staining agents such as, e.g., spaghetti sauce, and water-based staining agents such as, e.g., mustard. Preferably the fluoropolymeric orthodontic brackets exhibit a Delta E color shift of less than 2 (more preferably less than 1, and most preferably 0) when tested according to the hydrophilic color shift test procedure set forth below, and a Delta E color shift of less than 5 (more preferably less than 3, and most preferably 0) when tested according to the oleophilic color shift test procedure set forth below.

The orthodontic brackets appear translucent to transparent to the human eye such that when the article is placed on a surface such as, e.g., a tooth, the article transmits the color of the underlying surface through the article. Preferably the bracket exhibits at least about 0.001% transmittance at 546 nm, more preferably 0.01% transmittance at 546 nm when measured according to the Transmittance Test Procedure set forth below. Preferred articles exhibit a transmittance of at least about 0.001% over the wavelength range of 400 nm to 800 nm when measured according to the Transmittance Test Procedure.

The fluoropolymeric orthodontic brackets include fluoropolymer resins that are capable of being hardened and have sufficient strength, hydrolytic stability, and non-toxicity to render them suitable for use in the mouth. The fluoropolymers are translucent to transparent. Preferred fluoropolymers exhibit at least about 0.001% transmittance, more preferably 0.01 % transmittance, at 546 nm when measured according to the Transmittance Test Procedure. Useful fluoropolymers are perfluoroethylene-propylene copolymer, perfluoroalkoxyethylene, ethylene-tetrafluoroethylene copolymer, polychlrorotrifluoroethylene, ethylene-chlorotrifluoroethylene copolymer, or combinations thereof.

Examples of useful commercially available fluoropolymers include: Tefiel HT 2004 ethylene-tetrafluoroethylene copolymer including glass fibers, Tefzel 200, Tefzel 220, Tefzel 280, Tefzel 210, HT 2055, HT 2055NA, HT 2088NA, HT 2098, HT 2118, HT 2141, HT 2155 and HT 2158 ethylene-tetrafluoroethylene copolymers, and Teflon PFA 340 perfluoroalkoxy fluorocarbon resin, (all available from DuPont); Halar, Halar 300 and Halar 500 DA ethylene-chlorotrifluoroethylene copolymer (all available from Ausimont); and Kel-F81 PCTFE polychlorotrifluoroethylene (available from Minnesota Mining and Manufacturing),

Preferred adhesive systems for bonding a fluoropolymeric bracket to a tooth surface include a primer composition and a polymerizable component. The primer composition is capable of enabling or enhancing the adhesion of a fluoropolymeric bracket to the surface of a tooth. Useful primer compositions include an organoborane amine complex capable of initiating polymerization of a polymerizable component. The polymerizable component may include, e.g., monomers, oligomers, and combinations thereof that are capable of polymerizing and thereby forming an adhesive composition.

Preferred adhesive systems also include an effective amount of a compound that is reactive with or will remove the amine portion of the organoborane amine complex so as to liberate the organoborane and thereby allow the organoborane to initiate polymerization of polymerizable components.

Examples of useful organoborane amine complexes and adhesive systems that include an organoborane amine complex are described, e.g., in U.S. Patent Nos. 5,539,070 (Zharov et al.), 5,616,796 (Pocius et al.), 5,681,910 (Pocius), and 5,310,835 (Skoultchi et al.), and in WO 98/17694 and WO 97/07151

The fluoropolymeric orthodontic bracket the tooth, or a combination thereof, can be treated (i.e., primed) with a primer composition prior to adhering the bracket to the tooth. One example of a useful priming composition is Transbond XT Light cure priming composition (Minnesota Mining and Manufacturing ("3M"), St. Paul, MN). Other useful priming compositions are described in U.S. Patent No.5,558,516 (Horn et al.) The above-described organoborane amine adhesive compositions are also suitable priming compositions.

One example of a useful method for adhering the fluoropolymeric orthodontic bracket to the surface of a tooth includes contacting the orthodontic bracket with an adhesive composition and adhering the orthodontic bracket to the tooth surface. The method can include priming a surface, e.g., the surface of the tooth, the orthodontic bracket, and combinations thereof, with a priming composition prior to adhering the orthodontic bracket to the surface of the tooth. The surface can be primed with one or more priming compositions sequentially, simultaneously and in combinations thereof. In one preferred method, the surface is primed with a composition that includes an organoborane amine complex. In another preferred method, the surface can be pretreated, e.g., primed, and then packaged for later use as described, for example, in U.S. Patent No. 5,558,516 (Horn et al.) After pretreatment, the surface can be treated with a primer composition, an adhesive composition and combinations thereof.

## EXAMPLES

Transmittance Test Procedure

The % transmittance of light through an article is measured as follows. An article is placed in the well of a glass well slide such that the tooth contacting surface of the article is in contact with the slide. The well is filled with immersion liquid having a refractive index ("ND") of 1.515 and covered with a coverslip. The bracket containing slide is then placed under a microscope having a 3.2x objective, a numerical aperture ("N.A.") of 0.06, and a substage condensing lens system having a 0.3 N.A. The aperture and field diaphragms on the substage condensor are set up to produce the standard transparency by transmission measurement configuration (t(o/180)) of ASTM E-179-91a "Standard Guide for the Selection of Geometric Conditions for Measurement of Reflection and Transmission Properties of Materials.

The area of the article that is of interest is then brought into focus. The slide is then moved such that a reference area is brought into view (the focus of the microscope is not changed during this procedure). The reference area is an area within the well that contains immersion oil but does not contain the article. A calibration spectrum is collected from the reference area and is defined as having a transmittance of 1.0 over the wavelength range. The slide is then moved such that the area of interest of the article is brought into the field of view and the transmittance spectrum is collected over a predetermined wavelength or wavelength range e.g., 400 nm to 800 nm.

A series of measurements are taken at a number of locations on the article that are visible to an observer when the article is worn by a user, see, e.g., the numbers and letters indicated on FIGS. 3, 4, 5, 6 and 13. The measurements are taken in a labial-lingual direction along paths that extend from the labial-most surface of the article to the lingual-most base of the article, being careful to avoid areas in which a metal object is present in the article and would interfere with the path of the beam of radiation. The lowest transmittance value obtained for the article at a given wavelength is the transmittance of the article at that wavelength.

Color Measurement and Calculation Test Procedure

The color shift in an article is measured as follows. A LABSPHERE certified diffuse white reflectance standard (CSS-99-010 8812A) is placed on the stage of a Leitz Orthoplan Microscope equipped with a Leitz MPV-SP Spectrophotometer illuminated with a tungsten halogen lamp. The microscope is set to 3.2x objective with a numerical aperture ("N.A.") of 0.06. A fiber optic ring, which produces a 45 degree cone of illumination, is mounted on the objective lens of the microscope producing a standard 45° illumination/ 0° detection color measurement (R(45/0)) according to ASTM E-179-91a, "Standard Guide for the Selection of Geometric Conditions for Measurement of Reflection and Transmission Properties of Materials".

The spectrophotometer is set to a measuring spot of 1mm x 1mm, slit width of 6 nm, threshold of 1, high voltage of 480v, filter edge 100Hz, and integration 8x. Two scans are taken from 370 nm to 800 nm at a sampling rate of 1/3 nm and data is reported every 1 nm.

The lamp intensity of the halogen lamp is adjusted such that the signal is never greater than 90% of that needed to saturate the detector. A calibration spectrum is collected from the area and is defined as having a reflectivity of 100% over the wavelength range. The reflectance standard is removed and a sample is placed on the microscope stage. An area of interest is brought into crisp focus and a reflectance spectrum is collected. The reflectance standard is returned to the microscope stage, brought into focus and the reflectance spectrum is collected. If the spectrum collected is other than 100% for the second measurement of the standard over the wavelength range, the standardization steps are repeated.

The color measurements and calculations are performed in accordance with ASTM standards E-308-95, "Standard Practice for Computing the Colors of Objects by Using the CIE System", and D 2244-93, "Standard Test Method for Calculation of Color Differences from Instrumentally Measured Color Coordinates" using the L*a*b* color representation. The L* value is a measure of the brightness of the sample. The a* is a measure of the redness for a positive value or greenness for a negative value, and b* is a measure of yellowness for a positive value or blueness for a negative value.

Color differences (i.e., $\Delta$L*, $\Delta$a* and $\Delta$b*) are calculated by subtracting the reference value from the sample value. The total color shift is represented by delta E according to the following formula:

$$\Delta E = \sqrt{\Delta L*^2 + \Delta a*^2 + \Delta b*^2}$$

Hydrophilic Color Shift Test Procedure

The color shift exhibited by an article after contact with a hydrophilic substance is measured as follows. Specimens are immersed for one hour at 60°C in French's 100% pure prepared yellow mustard. The samples are then removed, rinsed thoroughly with water, and air-dried.

The samples are then tested according to the above Color Shift Measurement and Calculation Test Procedure to determine Delta E.

Oleophilic Color Shift Test Procedure

The color shift exhibited by an article after contact with an oleophilic substance is measured as follows. Specimens are immersed for 2.5 hours at 60°C in Ragu Old World Style Flavored Spaghetti sauce with meat. The samples are then removed, rinsed thoroughly with water, and air-dried. The samples are then tested according to the above Color Shift Measurement and Calculation Test Procedure to determine Delta E.

Staining Test Procedure

**Example 1**

A 1 cm x 1 cm x 3 mm sample of Ausimont PFA 450 perfluoroalkoxy fluorocarbon resin (Ausimont) was immersed in French's mustard for period of two weeks at 60°C. The sample was then removed, washed with water, dried and adhered onto a white paper chart. Visual observations regarding the degree of staining were made and recorded. The results are summarized in Table L

Additional samples of the Ausimont PFA 450 were immersed in either Ragu spaghetti sauce, chili powder (Spice Island) at 1g per 10g water, or curry powder (Shilling) at 1g per 10g water. The samples remained in the staining agents for two weeks at 60°C. After two weeks the samples were removed, washed with water, and adhered to a chart. Visual observations regarding the degree of staining were made and recorded. The results are summarized in Table I.

**Example 2**

1 cm x 1 cm x 3 mm samples of Ausimont Halar polyethylene-chlorotrifluoroethylene were stain tested as in Example 1. No staining was observed.

**Example 3**

1 cm x 1 cm x 3 mm samples of DuPont PFA 340 perfluoroalkoxy fluorocarbon resin (DuPont) were stain tested as in Example 1. No staining was observed.

**Example 4**

1 cm x 1 cm x 3 mm samples of Tefzel 210 ethylene-tetrafluomethylene (DuPont) were stain tested as in Example 1. No staining was observed for the samples immersed in spaghetti sauce, mustard, and chili. A very light yellow was observed for the sample immersed in curry.

**Example 5**

1 cm x 1 cm x 3 mm samples of Tefzel 280 ethylene-tetrafluoroethylene (DuPont) were stain tested as in Example 1. No staining was observed.

The results obtained in Examples 1-5 are summarized in Table I.

**TABLE I**

| Sample | Spaghetti Sauce | Mustard | Chili | Curry |
|---|---|---|---|---|
| Example 1 | None | None | None | None |
| Example 2 | None | None | None | None |
| Example 3 | None | None | None | None |
| Example 4 | None | None | None | v. Light yellow |
| Example 5 | None | None | None | None |

Bond Strength Test Method

Samples are adhered to a knurled steel ring and immersed in a 37°C water bath for 16-24 hours. The ring with bonded samples is removed from the water, air-dried, and then placed onto the test fixture of an Instron Universal tensile test equipment. A wire loop is placed into the upper jaw of the test fixture and through the sample such that it is affixed to the sample. The wire is then pulled by the upper jaw of the test fixture at a rate of 0.2 inches per minute (0.5 cm/min) at a full scale load of 100 lbs (45 kg). The bond strength (in pounds) at separation of the sample from the metal ring is recorded.

Primer Composition Preparation

A primer composition was prepared by combining:

3.5 parts by weight of a monomer mixture of 39 parts by weight methyl methacrylate, 28 parts by weight butyl acrylate, 3 parts by weight methacrylic acid, 30 parts by weight poly(methylmethacrylate-co-ethyl acrylate (mol. wt. 101,000 (Aldrich)), and 100 parts by weight of a complex of triethylborane and Dytek A 2-methyl-1,5-pentane diamine, having one equivalent of boron per equivalent of nitrogen, and mixing with a wooden stick.

Sample Preparation

**Example 6**

The 0.231 cm x 0.330 cm surface of a 0.231 cm x 0.330 cm x 0.183 cm embossed cube of Halar 500DA ethylene-chlorotrifluoroethylene copolymer (available from Ausimont U.S.A. Inc.) was dipped into the above-described primer composition such that the primer composition completely covered the surface. The primed surface was then pressed against a grooved, knurled steel bonding ring, such that the cube adhered to the bonding ring, and the primer composition was allowed to cure for 48 hours.

The cube was then tested according to the Bond Strength Test Method. The results are reported in Table II.

**Example 7**

A dab of primer composition prepared as described above was coated onto a polyimide film. The 0.231 cm x 0.330 cm surface of a 0.231 cm x 0.330 cm x 0.183cm embossed cube of Halar 500DA ethylene-chlorotrifluoroethylene copolymer (Ausimont) was dipped into the primer composition such that a face of the cube was completely covered with the primer composition. The primer composition was allowed to cure for 48 hours, after which the cube was peeled from the polyimide film. Transbond XT light cure primer composition (3M) was then applied to the primed surface of the cube. The Transbond XT primer was allowed to sit for 60 seconds and then cured with visible light. The primed surface was then coated with Transbond XT light cure adhesive composition (3M), and adhered to a knurled steel bonding ring. The Transbond XT adhesive was then cured by exposing the Transbond XT adhesive to radiation emitted from an Ortholux XT Visible Light Curing Unit for 10 seconds.

The bonded cube was then tested according to the Bond Strength Test Method. The results are reported in Table II.

**Example 8**

The 0.231 cm x 0.330 cm surface of a 0.231 cm x 0.330 cm x 0.183 cm cube of Halar 500DA ethylene-chlorotrifluoroethylene copolymer (Ausimont) was primed and adhered to a grooved, knurled steel ring according to the method set forth in Example 6.

The bonded cube was then tested according to the Bond Strength Test Method. The results are reported in Table II.

**Example 9**

The 0.231 cm x 0.330 cm surface of a 0.231 cm x 0.330 cm x 0.183 cm cube of Halar 500DA ethylene-chlorotrifluoroethylene copolymer (Ausimont) was primed and adhered to a grooved, knurled steel ring according to the method set forth in Example 7.

The bonded cube was then tested according to the Bond Strength Test Method. The results are reported in Table II.

**Example 10**

A base surface of a test sample in a form depicted in Figs. 5 and 6 made from Teflon PFA340 perfluoroalkoxy fluorocarbon resin (DuPont) was primed and adhered to a grooved, knurled steel ring according to the method set forth in Example 6. The test sample was then tested according to the Bond Strength Test Method. The results are reported in Table II.

**Example 11**

A base surface of a test sample in a form depicted in Figs. 5 and 6 made from Teflon PFA340 was primed and adhered to a grooved, knurled steel ring according to the method set forth in Example 7. The bonded bracket was then tested according to the Bond Strength Test Method. The results are reported in Table II.

For Examples 6-11, the bond strength ranged from 7 to 23 pounds ("lbs")/sample (3 to 10 $\mu$g/sample) with an average of 16 lbs/sample (7 $\mu$g/sample). The failure mode was adhesive between the primer composition and the sample (i.e., the cube or the test sample).

**TABLE II**

| Example | Bond Strength (lbs) / (kg) | Standard Deviation |
|---------|----------------------------|--------------------|
| 6 | 22 / 10 | 3.0 |
| 7 | 5 / 2 | 1.4 |
| 8 | 20 /9 | 5.4 |
| 9 | 10/5 | 3.9 |
| 10 | 13/6 | 5.3 |
| 11 | 6/3 | 2.7 |

**Example 12**

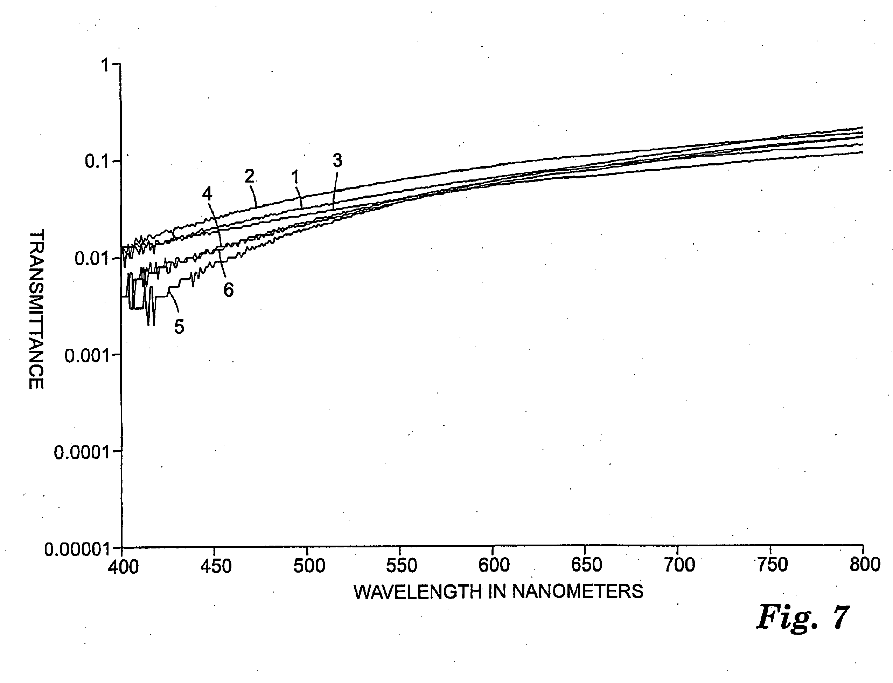

A test sample formed from virgin Halar ethytene-chiorotrinuoroethytene copolymer (Ausimont), having a shape as depicted in FIGS. 5 and 6, was tested according to the Transmittance Test Procedure set forth above. Transmittance was measured through regions identified as 1, 2, 3, 4, 5 and 6 in FIG. 5. The results are shown in % transmittance versus wavelength (nm) in FIG. 7.

**Example 13**

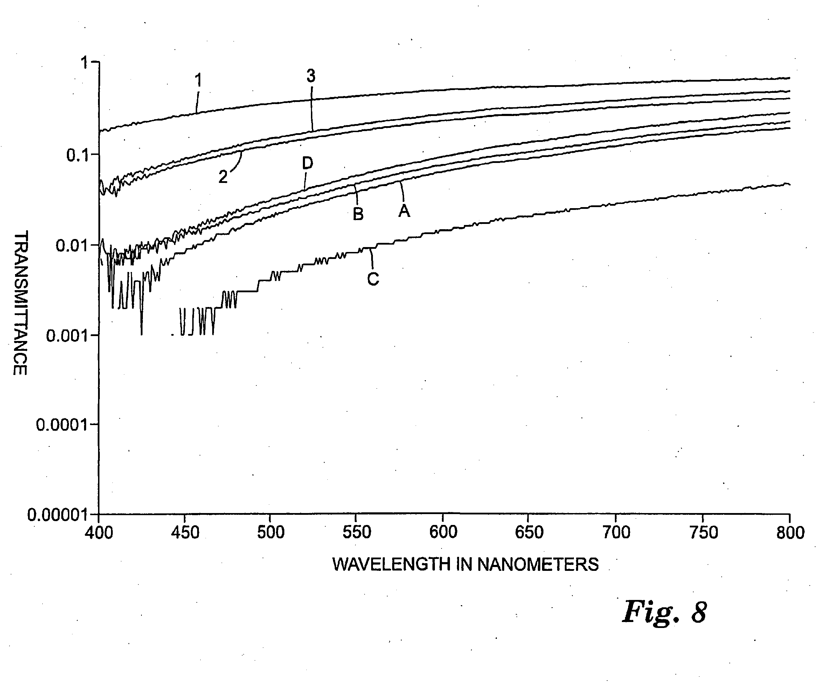

A bracket made from Halar ethylene-chlorotrifluoroethylene copolymer (Ausimont), having a shape as depicted in FIGS. 3 and 4, was tested according to the Transmittance Test Procedure set forth above. Transmittance data was obtained at locations identified as A, B, C, D, 1, 2 and 3 on FIG. 3. The results are shown in % transmittance versus wavelength (nm) in FIG. 8.

**Example 14** (Reference example)

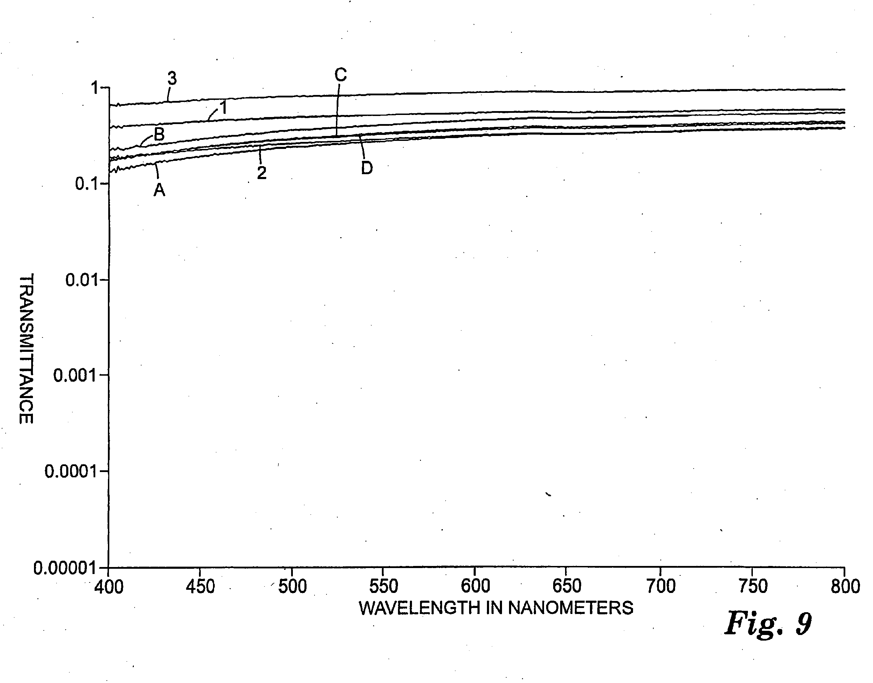

A bracket made from Teflon PFA tetrafluoroethylene-perfluorovinylether copolymer (DuPont), having a shape as depicted in FIGS. 3 and 4. was tested according to the Transmittance Test Procedure set forth above. Transmittance data was obtained at locations identified as A, B, C, D, 1, 2 and 3 in FIG. 3. The results are shown in % transmittance versus wavelength (nm) in FIG. 9.

**Example 15** (Reference example)

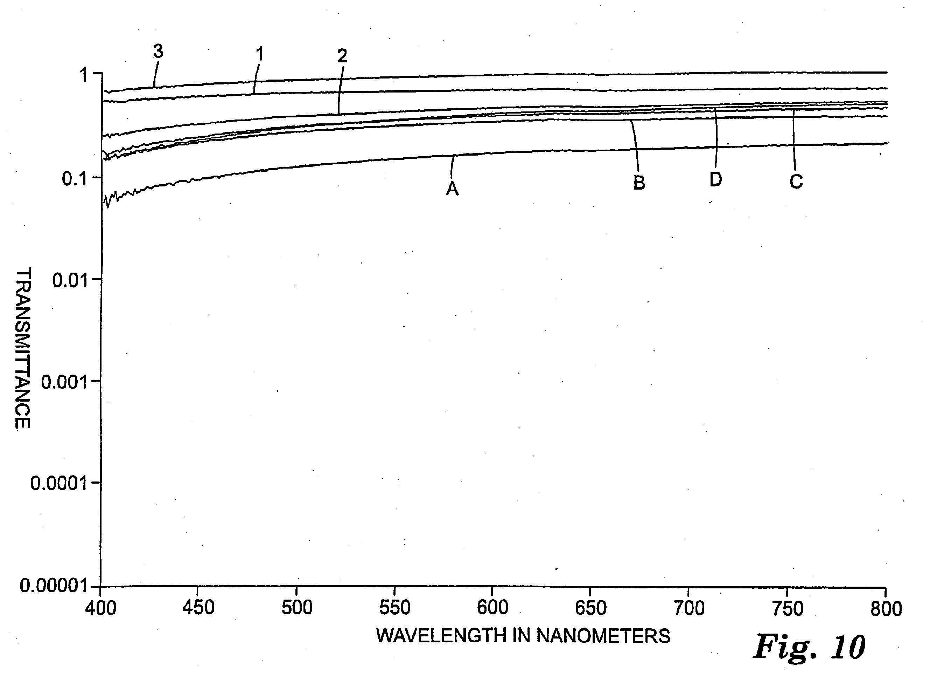

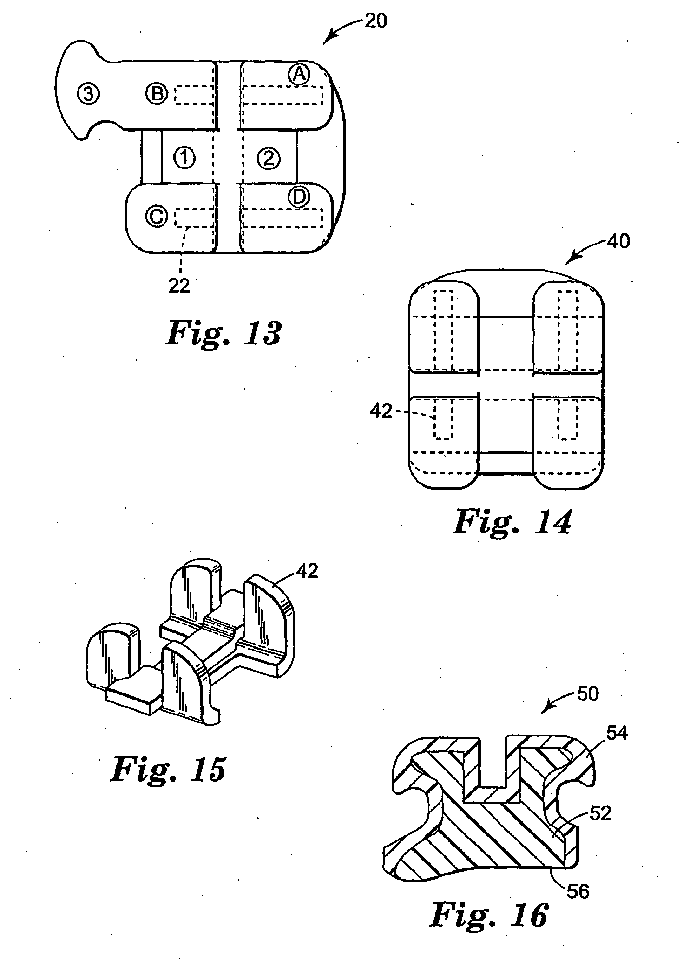

A bracket made from Teflon PFA tetraftuoroethylene-perfluorovinylether copolymer (DuPont) having a shape similar to the shape depicted in FIGS. 3 and 4 but with an embedded metallic framework as depicted in FIG. 13, was tested according to the Transmittance Test Procedure set forth above. Transmittance data was obtained at locations identified as A, B, C, D, 1, 2 and 3 in FIG. 13. The results are shown in % transmittance versus wavelength (nm) in FIG. 10.

**Example 16**

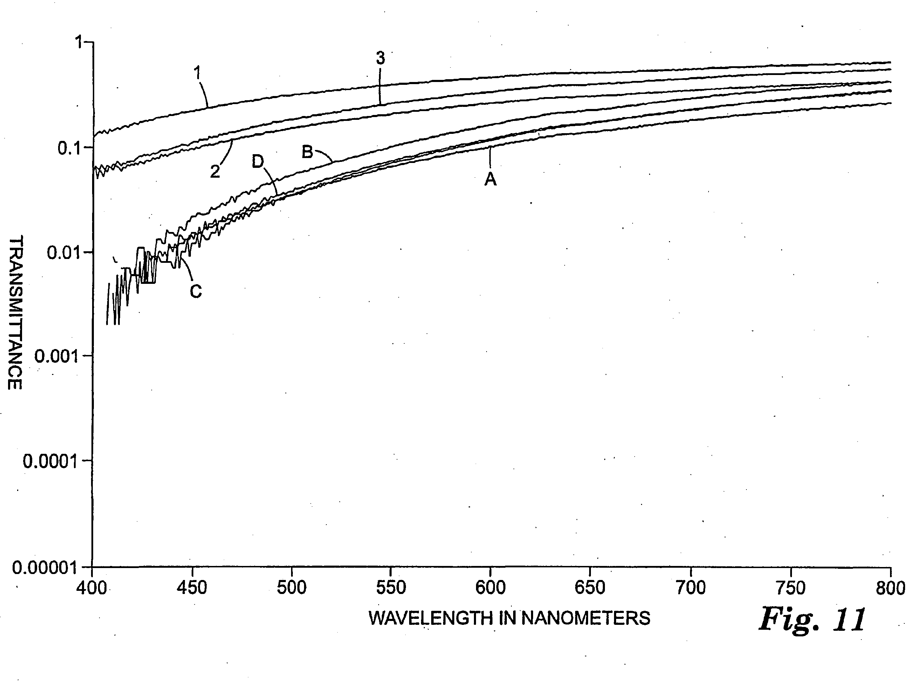

A bracket made from Tefzel 210 ethylene-tetrafluoroethylene copolymer (available from DuPont), having a shape as depicted in FIGS. 3 and 4, was tested according to the Transmittance Test Procedure set forth above. Transmittance data was obtained at locations identified as A, B, C, D, 1, 2 and 3 in FIG. 3. The results are shown in % transmittance versus wavelength (nm) in FIG. 11.

**Comparative Example 1**

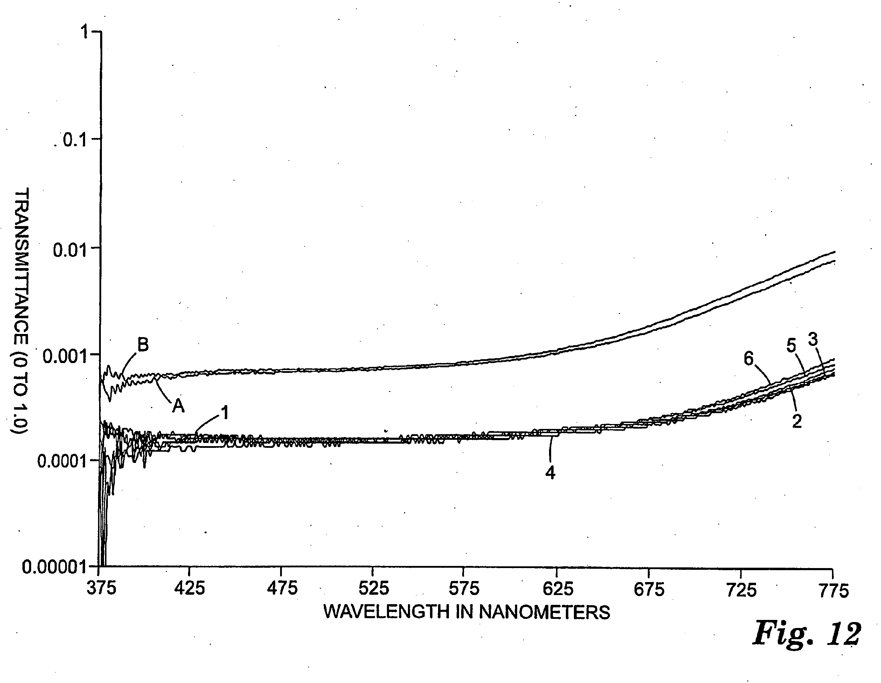

A test article made from Teflon® polytetrafluoroethylene (available from DuPont), having a shape as depicted in FIGS. 5 and 6, was tested according to the Transmittance Test Procedure set forth above. Transmittance data was obtained at locations identified as A, B, 1, 2, 3, 4, 5 and 6 in FIG. 5. The results are shown in % transmittance versus wavelength (nm) in FIG. 12.

Other embodiments are within the claims. For example, the bracket can be pretreated with a polymerization initiator including, e.g., the above-described organoborane amine complexes, and packaged in a suitable package, e.g., a kit. The bracket that have been pretreated with a polymerization initiator can also be provided with an adhesive precoat and packaged in a suitable package that optionally includes other components necessary to form an adhesive composition.

The bracket may include a metallic component, e.g., a framework. Orthodontic brackets 20 and 40 including a metal framework 22, 42 depicted by dotted lines are shown in FIGS. 13 and 14. One embodiment of a metallic framework 42 is shown in perspective view in FIG. 15. The metallic framework 22, 42 may be partially or wholly embedded in the body of the bracket 20,40. The framework can be coated or otherwise colored, e.g., by ink, paint, or porcelain, to match the color of the tooth or the color of the fluoropolymeric material. Examples of a metallic framework are described in U.S. Patent 5,597,302 (Pospisil et al.).

The bracket may also include a nonfluoropolymeric interior and a fluoropolymeric exterior layer. The fluoropolymeric exterior layer may extend over the entire surface of the nonfluoropolymeric interior or a portion thereof. Examples of suitable nonfluoropolymeric materials include glass, ceramic, plastic (e.g., polycarbonate and polyurethane), or a combination thereof. One example of such an article would include a polycarbonate bracket having an exterior fluoropolymeric layer, and, optionally, an exposed polycarbonate surface, which can be used for bonding the bracket to a tooth. Referring to FIG. 16, an orthodontic bracket 50 having a nonfluoropolymeric interior 52 and a fluoropolymeric layer 54 surrounding a portion of the nonfluoropolymeric interior 52 is shown. Surface 56 of nonfluoropolymeric interior 52 is exposed and is available for bonding to a tooth.

**Claims**

**1.** An orthodontic bracket comprising a fluoropolymer selected from the group consisting of perfluoroethylene-propylene copolymer, perfluoroalkoxyethylene, ethylene-tetrafluoroethylene copolymer, polychlorotrifluoroethylene, ethylene-chlorotrifluoroethylene copolymer, or a combination thereof.

2. The bracket of claim 1, wherein said bracket exhibits a Delta E color shift of no greater than about 2 when tested according to the Hydrophilic Color Shift Test, and a Delta E color shift of no greater than about 5 when tested according to the Oleophilic Color Shift Test.

3. The bracket of claim 1, wherein said fluoropolymer comprises perfluoroethylene-propylene copolymer, perfluoroalkoxyethylene, or ethylene-chlorotrifluoroethylene copolymer.

4. The bracket of claim 1, further comprising a polymeric composition disposed on a surface of said bracket, said polymeric composition comprising an organoborane compound.

5. The bracket of claim 1, further comprising an organoborane amine complex disposed on a surface of said bracket.

6. The bracket of claim 1, further comprising a metallic component.

7. A kit for adhering an orthodontic bracket to a tooth, said kit comprising:

> an orthodontic bracket as defined in any of claims 1 to 3, 6; and
> an adhesive system comprising

>> a) a polymerizable component; and
>> b) an organoborane amine complex.

8. The kit of claim 7, further comprising a, polyimide film.


**Patentansprüche**

1. Orthodontische Spange umfassend ein Fluorpolymer ausgewählt aus der Gruppe bestehend aus Perfluorethylen-Propylen-Copolymer, Perfluoralkoxyethylen, Ethylen-Tetrafluorethylen-Copolymer, Polychlortrifluorethylen, Ethylen-Chlortrifluorethylen-Copolymer oder einer Kombination derselben.

2. Spange nach Anspruch 1, wobei die Spange eine Delta E-Farbabweichung von nicht mehr als etwa 2 beim Testen gemäß dem hydrophilen Farbabweichungstest und eine Delta E-Farbabweichung von nicht mehr als etwa 5 beim Testen gemäß dem oleophilen Farbabweichungstest aufweist.

3. Spange nach Anspruch 1, wobei das Fluorpolymer Perfluorethylen-Propylen-Copolymer, Perfluoralkoxyethylen oder Ethylen-Chlortrifluorethylen-Copolymer umfasst.

4. Spange nach Anspruch 1, ferner umfassend eine polymere Zusammensetzung, die auf einer Oberfläche der Spange aufgebracht ist, wobei die polymere Zusammensetzung eine Organoboranverbindung umfasst.

5. Spange nach Anspruch 1, ferner umfassend einen Organoboranaminkomplex, der auf einer Oberfläche der Spange aufgebracht ist.

6. Spange nach Anspruch 1, ferner umfassend eine Metallkomponente.

7. Kit für das Befestigen einer orthodontischen Spange an einem Zahn, wobei der Kit umfasst:

> eine orthodontische Spange, wie in einem der Ansprüche 1 bis 3 und 6 definiert; und
> ein Klebstoffsystem, umfassend:

>> a) eine polymerisierbare Komponente; und
>> b) einen Organoboranaminkomplex.

8. Kit nach Anspruch 7, ferner umfassend eine Polyimidfolie.

**Revendications**

1. Bracket orthodontique comprenant un fluoropolymère sélectionné dans le groupe constitué d'un copolymère de perfluoroéthylène-propylène, du perfluoroalkoxyéthylène, d'un copolymère d'éthylène-tétrafluoroéthylène, du polychlorotrifluoroéthylène, d'un copolymère d'éthylène-chlorotrifluoroéthylène, ou d'une combinaison de ceux-ci.

2. Bracket selon la revendication 1, ledit bracket présentant une distorsion chromatique Delta E ne dépassant pas environ 2 quand celle-ci est évaluée selon l'Essai de distorsion chromatique hydrophile, et une distorsion chromatique Delta E ne dépassant pas environ 5 quand celle-ci est évaluée selon l'Essai de distorsion chromatique oléophile.

3. Bracket selon la revendication 1, dans lequel ledit fluoropolymère comprend un copolymère de perfluoroéthylène-propylène, le perfluoroalkoxyéthylène, ou un copolymère d'éthylène-chlorotrifluoroéthylène.

4. Bracket selon la revendication 1, comprenant en outre une composition polymère disposée sur une surface dudit bracket, ladite composition polymère comprenant un composé organoborane.

5. Bracket selon la revendication 1, comprenant en outre un complexe amine organoborane disposé sur une surface dudit bracket.

6. Bracket selon la revendication 1, comprenant en outre un composant métallique.

7. Kit pour faire adhérer un bracket orthodontique à une dent, ledit kit comprenant :

   un bracket orthodontique comme il est défini dans l'une quelconque des revendications 1 à 3 et 6 ; et
   un système adhésif comprenant :

   a) un composant polymérisable ; et
   b) un complexe amine organoborane.

8. Kit selon la revendication 7, comprenant en outre un film de polyimide.

**Fig. 1**

**Fig. 2**

**Fig. 3**

*Fig. 4*

*Fig. 5*

*Fig. 6*

Fig. 7

**TRANSMITTANCE** (y-axis)

**WAVELENGTH IN NANOMETERS** (x-axis)

Curves labeled: 1, 3, 2, D, B, A, C

*Fig. 8*

EP 1 278 503 B1

Fig. 9

EP 1 278 503 B1

*Fig. 10*

EP 1 278 503 B1

*Fig. 11*

Fig. 12

*Fig. 13*

*Fig. 14*

*Fig. 15*

*Fig. 16*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2253420 A
- US 3545083 A
- WO 9522567 A
- EP 498558 A
- US 5539070 A, Zharov
- US 5616796 A, Pocius
- US 5681910 A, Pocius
- US 5310835 A, Skoultchi
- WO 9817694 A
- WO 9707151 A
- US 5558516 A
- US 5597302 A, Pospisil